# DEMANDE DE BREVET EUROPEEN

(11) **EP 2 184 011 A1**
(43) Date de publication de la demande: **12.05.2010**
(21) Numéro de dépôt: 09172836.0
(22) Date de dépôt: 13.10.2009
(51) Int. Cl.: A61B 5/08, A61N 1/36, A61N 1/365

(54) **Dispositif médical de type prothèse cardiaque implantable, intégrant des moyens spirométriques pour le diagnostic de pathologies pulmonaires**

(30) Priorité: 06.11.2008 FR 0806199
(71) Demandeur: ELA MEDICAL, 92541 Montrouge (FR)
(72) Inventeur: Casset, Cyrille, 33650, SAINT SELVE (FR)
(74) Mandataire: Dupuis-Latour, Dominique

(57) **Abrégé**

Ce dispositif médical implantable actif comprend des moyens de mesure de l'activité respiratoire du patient, pour recueillir un signal d'impédance transthoracique fonction des variations instantanées du volume pulmonaire. Il comprend en outre des moyens spirométriques pour produire, à partir du signal d'impédance transthoracique recueilli sur un cycle respiratoire, des couples de valeurs débit (dV/dt) /volume (V) pulmonaire représentatifs d'une caractéristique spirométrique (S) du patient. Des moyens d'analyse permettent de déduire de la caractéristique spirométrique produite au moins un paramètre d'état pulmonaire du patient et de comparer celui-ci à une valeur de référence prédéterminée, pour produire un indicateur diagnostique fonction du résultat de cette comparaison.

## Description

L'invention concerne le diagnostic des troubles respiratoires chez des patients appareillés avec une prothèse implantée de type "dispositif médical actif implantable", c'est-à-dire un dispositif tel que défini par la Directive 90/385/CEE du 20 juin 1990 du Conseil des communautés européennes, notamment un implant cardiaque apte à délivrer si nécessaire au coeur des impulsions électriques de stimulation, de resynchronisation, de cardioversion et/ou de défibrillation en cas de trouble du rythme détecté par le dispositif.

Ces dispositifs peuvent être pourvus de moyens de mesure de l'activité respiratoire du patient porteur de l'implant.

Cette mesure est typiquement une mesure transthoracique d'impédance, opérée entre deux électrodes disposées dans la cage thoracique ou entre le boîtier du dispositif et une électrode distante, par exemple une électrode de stimulation (dans le cas où l'implant est un stimulateur cardiaque). L'impédance est mesurée par injection d'un courant de quelques centaines de microampères à une fréquence de quelques hertz. Les variations de l'impédance instantanée ainsi mesurées reproduisent les variations du volume thoracique, avec des minima d'impédance quand les poumons sont remplis d'air, et une impédance croissante lors de la phase expiratoire.

Ce signal d'impédance est habituellement utilisé pour produire une indication représentative de la "ventilation-minute" (volume x fréquence respiratoire), après moyennage sur quelques dizaines de cycles respiratoires. Le signal de ventilation-minute est un bon indicateur des besoins métaboliques courants du patient, et sert en particulier pour asservir en fréquence un stimulateur cardiaque, par exemple pour augmenter la fréquence de stimulation en cas d'augmentation des besoins métaboliques.

Le US 2006/0020295 A1 décrit un tel dispositif, qui opère une caractérisation de la courbe décrivant la ventilation dans le domaine temporel en rapport avec l'activité du patient, notamment à partir de l'amplitude pic à pic (*tidal volume*) et de la fréquence respiratoire (*respiratory rate*).

Le US 7 329 226 B1 décrit un dispositif d'un type comparable au précédent, qui analyse des profils ventilatoires en distinguant notamment les phases inspiratoires et les phases expiratoires qui alternent au cours du temps.

Le signal d'impédance transthoracique peut également être utilisé pour détecter et diagnostiquer des pathologies respiratoires, par exemple pour détecter des situations d'apnée ou d'hypopnée dans le cadre de la recherche d'un syndrome d'apnée du sommeil (SAS). Les variations du rythme respiratoire sont analysées de manière à détecter la survenue d'apnées ou d'hypopnées et d'en évaluer la récurrence, plus ou moins importante, sur une période prolongée de plusieurs heures (durée d'une phase de sommeil), voire sur plusieurs jours pour évaluer la manière dont évolue le syndrome. Le EP 0 970 713 A1 décrit un dispositif pourvu de tels moyens de diagnostic du SAS à partir d'un signal d'impédance transthoracique. D'autres pathologies, par exemple le syndrome dit de "Cheyne-Stokes" caractéristique de patients souffrant d'insuffisance cardiaque, peuvent être détectées en analysant, par un suivi du signal d'impédance transthoracique, la manière dont varie l'activité respiratoire sur plusieurs cycles successifs, comme cela est exposé par exemple dans le EP 1 295 623 A1 (ELA Medical).

Le point de départ de l'invention réside dans la constatation qu'il est possible d'utiliser le signal d'impédance transthoracique non seulement pour l'asservissement d'un stimulateur ou pour la recherche de syndromes respiratoires apparaissant sur plusieurs cycles, mais également pour l'obtention de données spirométriques.

À la différence des dispositifs connus tels que ceux cités plus haut qui cherchent à caractériser la courbe de ventilation par son évolution dans le domaine temporel, l'analyse spirométrique repose sur l'analyse d'une courbe tracée à partir de données représentatives des volumes et des débits d'air (*air flow rates*) instantanés pendant un cycle respiratoire donné, ce cycle respiratoire étant en principe un cycle unique, isolé, pour lequel on a demandé au patient d'inspirer et expirer au maximum de ses capacités.

L'analyse spirométrique est en elle-même bien connue. Elle est réalisée en demandant au patient d'effectuer une inspiration et une expiration profondes dans un appareil spécifique (spiromètre) assurant la mesure en temps réel à la fois des débits et des volumes d'air inspirés et expirés pendant cette épreuve. Le résultat peut être représenté sous forme d'une caractéristique débit/ volume (ci-après "caractéristique" ou "courbe"), et un praticien peut tirer de très nombreuses informations utiles par un simple examen visuel de la morphologie de cette courbe.

On notera qu'à la différence des techniques évoquées plus haut reposant sur l'analyse du signal de ventilation, la dimension temporelle n'intervient pas dans l'analyse spirométrique.

L'un des buts de l'invention est de permettre une telle analyse spirométrique chez des patients appareillés avec une prothèse munie de moyens de mesure d'impédance transthoracique (typiquement, des moyens destinés à évaluer la ventilation-minute), sans utilisation d'un spiromètre ni d'aucun appareillage externe supplémentaire.

L'absence de recours à tout appareil externe permet de simplifier les mesures, qui peuvent être réalisées en toute circonstance et en tout lieu, et même de façon automatique si l'on souhaite effectuer un suivi du patient en monitorant les informations issues de l'analyse spirométrique.

Un autre but de l'invention est de pouvoir effectuer cette mesure de façon purement autonome, sans calibration préalable ni nécessité d'une référence externe.

À cet égard, si l'on peut conserver tout ou partie de certaines données pertinentes issues d'une mesure spirométrique et monitorer de manière régulière ou permanente ces données, il sera possible d'informer le médecin de façon la plus complète lors d'une visite ou même, sans attendre cette visite, alerter en cas de besoin le médecin ou le patient, ou encore modifier une ou plusieurs des thérapies délivrées au patient, notamment les thérapies de stimulation et de resynchronisation appliquées par l'implant lui-même.

L'intérêt d'un tel suivi est d'autant plus souhaitable que, si l'on considère la population de patients atteints d'insuffisance cardiaque, le comportement de l'appareil respiratoire est fortement impacté par la décompensation cardiaque consécutive à la dégradation de la fonction cardiaque. L'un des processus principaux consiste en une élévation des pressions sanguines au niveau pulmonaire ayant pour effet de remplir les alvéoles pulmonaires d'eau afin de s'opposer à cette pression (le poumon remplace l'air par de l'eau afin d'augmenter la pression côté poumons pour s'opposer à la pression côté sang). Ceci a pour conséquence de réduire la capacité pulmonaire des patients, rendant la respiration de plus en plus difficile et l'oxygénation du sang de plus en plus mauvaise. L'augmentation de la fréquence et du volume respiratoire est donc un effet de cette insuffisance cardiaque. *A contrario,* une amélioration durable de l'état cardiaque aura les effets inverses, eux aussi visibles sur le signal ventilatoire.

La population de patients souffrant d'insuffisance cardiaque (dont beaucoup sont des patients déjà équipés d'un implant) est de ce fait une cible privilégiée pour une analyse et un suivi d'informations sur la ventilation pulmonaire issues d'une analyse spirométrique.

En second lieu, au-delà de la stimulation cardiaque, l'analyse spirométrique peut permettre un dépistage précoce et un suivi des patients atteints de bronchopneumopathie chronique obstructive (BPCO). La BPCO est une pathologie grave, cause importante de décès, avec comorbidité fréquente chez les patients insuffisants cardiaques (les études épidémiologiques suggèrent en effet une forte association entre la présence d'une BPCO et la présence d'une cardiopathie ischémique, qui est l'une des causes principales d'insuffisance cardiaque). Cette pathologie - qui n'est pas réversible - impose un suivi continu des patients, d'autant plus nécessaire qu'elle présente des évolutions doubles : lentes et continues, ou bien rapides par crises brutales de décompensation ventilatoire.

L'un des buts de l'invention est, à cet égard, de proposer un moyen permettant un suivi de l'état pulmonaire de patients atteints de BPCO, de manière précise (par exemple quotidienne) et sans acte médical. Un second intérêt est d'informer rapidement le praticien en cas d'évolution importante de la pathologie, par exemple par une technique de *"home monitoring"* avec téléalerte et télétransmission des données, de façon à réduire le laps de temps séparant le début de la dégradation de la prise en charge du patient (qui, généralement, ne prend conscience de la gravité de son état que tardivement, bien après le début de la dégradation).

L'invention propose à cet effet un dispositif médical implantable actif, comprenant des moyens de mesure de l'activité respiratoire du patient, aptes à recueillir un signal d'impédance transthoracique fonction des variations instantanées du volume pulmonaire.

De façon caractéristique de l'invention, ce dispositif comprend des moyens spirométriques, aptes à produire, à partir du signal d'impédance transthoracique recueilli sur au moins un cycle respiratoire, des couples de valeurs formés à partir, d'une part, du volume pulmonaire instantané et, d'autre part, du débit pulmonaire instantané, représenté par la dérivée dudit volume pulmonaire, pour produire à partir de ces couples de valeurs une caractéristique spirométrique définie dans un espace débit vs. volume sans dimension temporelle.

De préférence, le dispositif comprend en outre :
- des moyens d'analyse de la caractéristique spirométrique produite, aptes à déduire de cette caractéristique au moins un paramètre d'état pulmonaire du patient ;
- des moyens de diagnostic associés aux moyens d'analyse et aptes à opérer une comparaison entre, d'une part, une valeur de référence prédéterminée et, d'autre part, ledit paramètre d'état pulmonaire ou la variation dans le temps dudit paramètre d'état pulmonaire, et à produire un indicateur diagnostique en fonction du résultat de cette comparaison ;
- des moyens de recherche de points remarquables de la caractéristique spirométrique, et/ou de modélisation linéaire ou curviligne de la caractéristique spirométrique ;
- des moyens de validation *a priori,* aptes à déterminer l'existence d'au moins une condition d'état prédéterminée (notamment une plage de valeurs de fréquence cardiaque du patient, un état de repos du patient, et/ou une plage horaire) lors du recueil du signal d'impédance transthoracique, et à inhiber la mise en oeuvre des moyens spirométriques en cas d'absence de ladite condition d'état ;
- des moyens de validation *a posteriori,* aptes à déterminer la conformité, ou non, de la caractéristique produite par les moyens spirométriques à au moins un critère prédéterminé (notamment le caractère fermé de la courbe décrivant la caractéristique spirométrique sur un cycle respiratoire, et/ou la stabilité de la caractéristique spirométrique sur une pluralité de cycles respiratoires successifs), et à inhiber la mise en oeuvre des moyens de diagnostic en cas de non-conformité ; et/ou
- des moyens d'activation sélective des moyens spirométriques, opérant en réponse à un signal de commande externe (notamment un ordre externe transmis au dispositif par télémétrie, ou une séquence de cycles respiratoires spécifiques produits par le patient et détectés par les moyens spirométriques).

On va maintenant décrire un exemple de mise en oeuvre du dispositif de l'invention, en référence aux dessins annexés.
La Figure 1 est un exemple de caractéristique spirométrique montrant la forme de la courbe obtenue pendant un cycle complet respiratoire.
La Figure 2 est un organigramme simplifié montrant les différentes étapes d'un enregistrement dans le cadre d'un suivi respiratoire.
La Figure 3 est un organigramme simplifié montrant les différentes étapes de l'enregistrement d'une respiration forcée demandée par un praticien lors d'une visite.
La Figure 4 est un organigramme simplifié montrant les différentes étapes de l'enregistrement d'une respiration forcée, déclenché par le patient lui-même.

On va maintenant décrire un exemple de réalisation du dispositif de l'invention.

En ce qui concerne ses aspects logiciels, l'invention peut être mise en oeuvre par une programmation appropriée du logiciel de commande d'un dispositif connu, par exemple un dispositif de stimulation, resynchronisation, cardioversion et/ou défibrillation, comprenant des moyens d'acquisition d'un signal fourni par des sondes endocavitaires et/ou un ou plusieurs capteurs implantés.

L'invention peut notamment être appliquée aux dispositifs implantables commercialisés par ELA Médical, Montrouge, France tels que les appareils *Symphony , Rhapsody* ou *Reply.*

Il s'agit de dispositifs à microprocesseur programmable comportant des circuits pour recevoir, mettre en forme et traiter des signaux électriques recueillis par des électrodes implantées, et délivrer des impulsions de stimulation à ces électrodes. Il est possible d'y transmettre par télémétrie des logiciels qui seront conservés en mémoire et exécutés pour mettre en oeuvre les fonctions de l'invention qui seront décrites ci-dessous. L'adaptation de ces appareils à la mise en oeuvre des fonctions de l'invention est à la portée de l'homme du métier, et elle ne sera pas décrite en détail.

Ces appareils sont en particulier pourvus de moyens de mesure d'impédance transthoracique, fonctionnant sur le principe exposé au début de la présente description.

Dans ces appareils, cette mesure est exploitée en dérivant un signal de "ventilation-minute" à partir de l'amplitude de crête et de la période du signal d'impédance transthoracique, ce signal étant ensuite utilisé pour asservir le stimulateur.

Dans le cadre de la présente invention, ce ne sont pas ces paramètres (amplitude de crête et période) qui sont utilisés, mais la variation complète du signal sur l'ensemble d'un cycle respiratoire, et ceci en principe sur la durée d'un seul cycle (sauf pour les contrôles préalables de stabilité des cycles respiratoires ou pour l'élimination de cycles artéfactés, par exemple).

La spirométrie est en effet une technique basée sur l'analyse d'une caractéristique relevée sur un cycle inspiration-expiration isolé, en principe sans moyennage (à l'opposé des mesures de ventilation-minute, qui ne peuvent donner d'indication significative que sur une étendue de plusieurs cycles ou dizaines de cycles respiratoires).

Si un certain nombre de conditions préalables (que l'on exposera plus bas) sont réunies, l'appareil effectue un enregistrement du signal d'impédance transthoracique Z(t) sur au moins un cycle respiratoire.

Le signal respiratoire étant cyclique, le dispositif détecte automatiquement un cycle respiratoire comme étant défini par le signal présent entre deux sommets successifs de la courbe Z(t). Des contrôles complémentaires peuvent être éventuellement ajoutés pour confirmer ou infirmer le cycle respiratoire enregistré, en vérifiant par exemple que l'amplitude de crête en fin de cycle est égale, à ± 5 % près, à celle en début de cycle. Il est également possible de mettre en oeuvre diverses techniques d'élimination de cycles considérés comme artéfactés (du fait d'un saut d'impédance statique, d'interférences entre rythme cardiaque et rythme respiratoire lorsque ceux-ci sont proches, etc.), comme cela est notamment décrit dans le EP 1 584 288 A1 (ELA Medical).

Le signal d'impédance, qui reflète les variations instantanées du volume pulmonaire, est enregistré et conservé dans la mémoire de l'appareil. Une fois ce signal obtenu, l'appareil calcule pour chaque point t du signal Z(t) échantillonné la valeur dZ(t)/dt, c'est-à-dire la valeur dérivée du signal, représentative du débit pulmonaire instantané dV/dt. Les couples de valeurs {Z(t), dZ(t)/dt}, qui sont représentatifs des valeurs débit/volume pulmonaire instantané au cours d'un cycle respiratoire donné, sont conservés dans la mémoire de l'appareil pour l'ensemble du cycle respiratoire.

L'enregistrement du signal d'impédance transthoracique recueilli peut être soumis à un certain nombre de conditions préalables telles que :
- plage horaire, en particulier lorsque l'on souhaite effectuer un enregistrement à intervalles réguliers d'un cycle respiratoire nocturne non forcé, dans le cadre d'un suivi de l'état pulmonaire du patient,
- condition de fréquence cardiaque (typiquement F_{c} < 70 bpm), et/ou
- condition d'activité du patient, si le dispositif est équipé d'un détecteur d'activité (capteur d'accélération G, notamment).

L'enregistrement qui a été ainsi opéré peut également faire l'objet d'une validation *a posteriori,* par exemple n'être conservé que si certains critères sont vérifiés, critères tels que : obtention d'une courbe fermée, stabilité des cycles respiratoires (dans le cas de cycles respiratoires non forcés), etc.

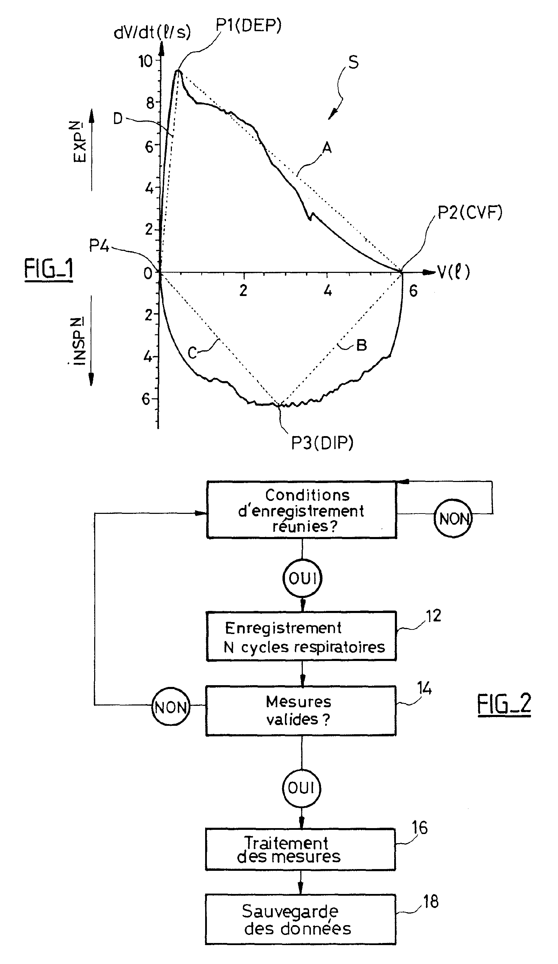

Les variations des couples de données {Z(t), dZ(t)/dt} peuvent être représentées graphiquement par une courbe S illustrée figure 1, donnant les variations du débit (dV/dt) en fonction du volume pulmonaire (V).

Cette courbe est produite au cours d'un cycle respiratoire et parcourue à partir de l'origine, au point d'intersection des deux axes. Lors de l'expiration, le débit augmente très rapidement et atteint son maximum après 100 ms environ, le point correspondant (nommé débit expiratoire de pointe DEP) étant référencé P1. La courbe redescend ensuite de façon à peu près régulière, avec une baisse du débit jusqu'à ce qu'il n'y ait plus de volume d'air expiré. Le point P2 correspondant à la fin de l'expiration est appelé capacité vitale forcée (CVF). Le reste de la courbe correspond à la phase inspiratoire (débit négatif), avec le point caractéristique P3 (débit inspiratoire de pointe, DIP) et retour à l'origine (point P4).

La courbe illustrée correspond à celle obtenue typiquement pour un patient sain à qui on demande d'exécuter une expiration et une inspiration forcées.

Dans le cas d'un patient souffrant de BPCO, la courbe présentera une forme différente, avec notamment une forme beaucoup plus concave de la phase expiratoire (arc P1P2), cela sans modification substantielle des valeurs DEP et CVF. En effet, en cas de BPCO, l'air des voies respiratoires hautes peut être expiré sans problème (le DEP apparaîtra donc normal). Mais après l'expiration d'air des voies hautes, l'air sortira des petites voies ; or dans le cas d'un syndrome obstructif, ces voies sont partiellement obstruées, et l'air sortira donc moins vite, le débit sera donc diminué, donnant la forme concave caractéristique (et cela bien que le patient ait souvent une CVF apparemment normale).

Une méthode d'analyse automatique de la caractéristique spirométrique consiste par exemple à suivre l'évolution de la position des points P1, P2, P3 et P4 au cours du temps, ainsi que la morphologie des arcs reliant ces points.

La caractéristique obtenue et mémorisée peut être simplifiée afin de ne conserver que les points remarquables (notamment les points P1, P4), et en modélisant les arcs curvilignes par exemple sous forme de quatre segments de droite A, B, C et D représentés en pointillés sur la figure 1. Une autre approche consiste à trouver une forme simple, par exemple elliptique, constituant la meilleure approximation de la courbe spirométrique ; l'analyse portera alors sur l'orientation et la dimension des axes principaux de cette ellipse.

Les évolutions de ces divers paramètres au cours du temps donneront des indications sur l'amélioration ou la dégradation de l'état ventilatoire du patient. Par exemple :
- un décalage de la courbe vers la droite au cours de plusieurs jours indiquera une dégradation potentielle de l'état ventilatoire ;
- une élévation du point P1 et un abaissement du point P3 (abaissement en valeur arithmétique, c'est-à-dire augmentation en valeur absolue) correspondront à une augmentation des débits maximaux d'inspiration et d'expiration, donc à une amélioration théorique de l'état ventilatoire général ;
- une réduction de l'écart entre les points P2 et P4 pourra indiquer une réduction du volume respiratoire exploité, donc une dégradation de l'état ventilatoire.

On pourra par exemple considérer qu'une variation de plus ou moins 10 % d'un ou plusieurs de ces paramètres sur une semaine sera révélatrice d'une modification significative de l'état du patient, et que des actions particulières pourront alors être entreprises : déclenchement d'une alerte, modification du traitement clinique, etc.

Par exemple, en cas de modification avérée de l'état respiratoire, le dispositif pourra décider de stopper l'application d'un mode spécifique de stimulation au profit d'un autre. Ainsi, dans le cas d'une stimulation double chambre, le dispositif pourra laisser le rythme ventriculaire spontané du patient s'exprimer afin de réduire le taux de stimulation ventriculaire.

Ou encore, une fonctionnalité comme la fréquence de repos pourra être désactivée en cas de dégradation de l'état ventilatoire enregistré en état basal (nocturne). En effet, un moyen de réduire l'impact de cette dégradation consiste à augmenter volontairement la fréquence cardiaque.

Une autre application possible est la modification de la fonction d'asservissement à la ventilation-minute, par inhibition de l'utilisation du capteur de ventilation afin de permettre au coeur de battre à un rythme plus soutenu.

On va maintenant décrire plusieurs techniques de mise en oeuvre de l'invention, en référence aux organigrammes des Figures 2 à 4.

La Figure 2 illustre un mode de mise en oeuvre dans lequel le dispositif opère un suivi régulier de la fonction ventilatoire du patient sur la base de cycles respiratoires standard (non forcés).

L'objectif est de fournir un suivi de la courbe débit/volume respiratoire courant au praticien lors des visites de routine. L'exploitation des données peut être effectuée par l'implant ou par le programmateur du praticien lors de la lecture des données enregistrées.

Si un certain nombre de conditions d'enregistrement sont réunies (étape 10), par exemple des conditions de rythme cardiaque, de plage horaire, d'état et d'activité du patient, le dispositif déclenche automatiquement l'enregistrement d'un ou plusieurs cycles respiratoires (étape 12). L'intervalle d'enregistrement est typiquement compris entre un jour et une semaine.

Le dispositif contrôle ensuite la qualité des cycles respiratoires successifs (étape 14). Si les cycles sont exploitables (validation *a posteriori* explicitée plus haut), le cycle est enregistré, analysé (étape 16) et sauvegardé (étape 18) dans la mémoire de l'implant pour lecture et interprétation ultérieures par un praticien.

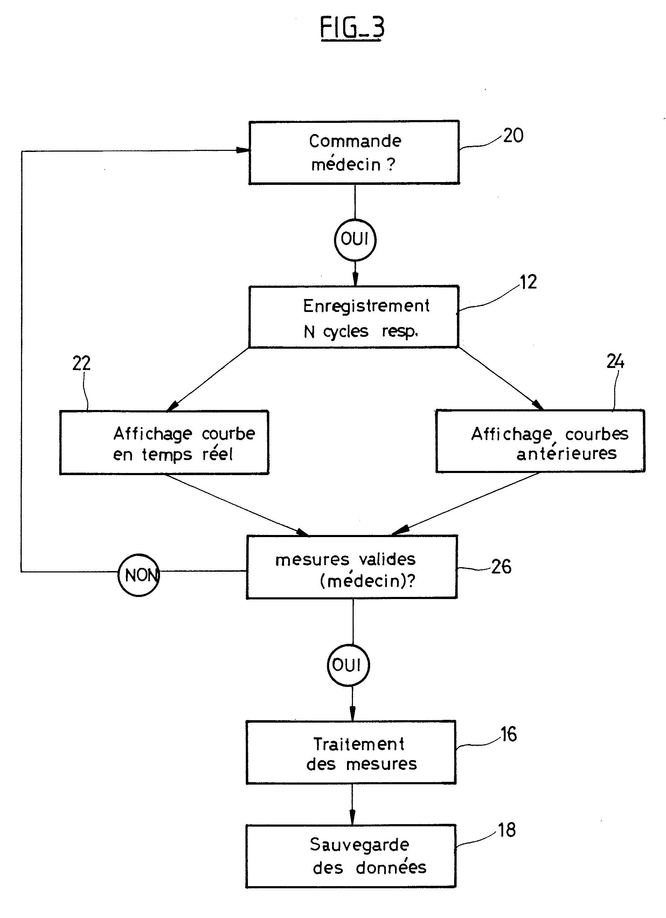

La Figure 3 illustre un autre mode de mise en oeuvre, lors d'une visite chez le praticien.

Ici, c'est le praticien qui déclenche l'enregistrement et demande au patient d'effectuer un cycle inspiration/expiration forcée. Le déclenchement de l'enregistrement (étape 20) peut se faire depuis le programmateur du médecin, par télémétrie. Le dispositif enregistre alors, comme précédemment, un ou plusieurs cycles respiratoires (étape 12), ce cycle étant affiché au praticien (étape 22) ainsi que les enregistrements antérieurs (étape 24). Le praticien peut de lui-même décider de conserver le cycle respiratoire enregistré (étape 26), de réitérer la mesure, ou de moyenner la courbe sur plusieurs cycles forcés. Les données sont alors, comme précédemment, traitées (étape 16) et enregistrées (étape 18).

L'intérêt de ce type d'enregistrement réside dans la possibilité d'effectuer des mesures tout à fait comparables à celles effectuées avec un spiromètre traditionnel (mesure de CVF, VEMS, etc.) sans que le médecin n'ait besoin d'un tel instrument, ou même des mesures effectuées en dehors de son cabinet, le seul appareillage nécessaire au praticien étant le programmateur, qu'il utilisera de toute façon pour lire les informations enregistrées dans l'implant du patient.

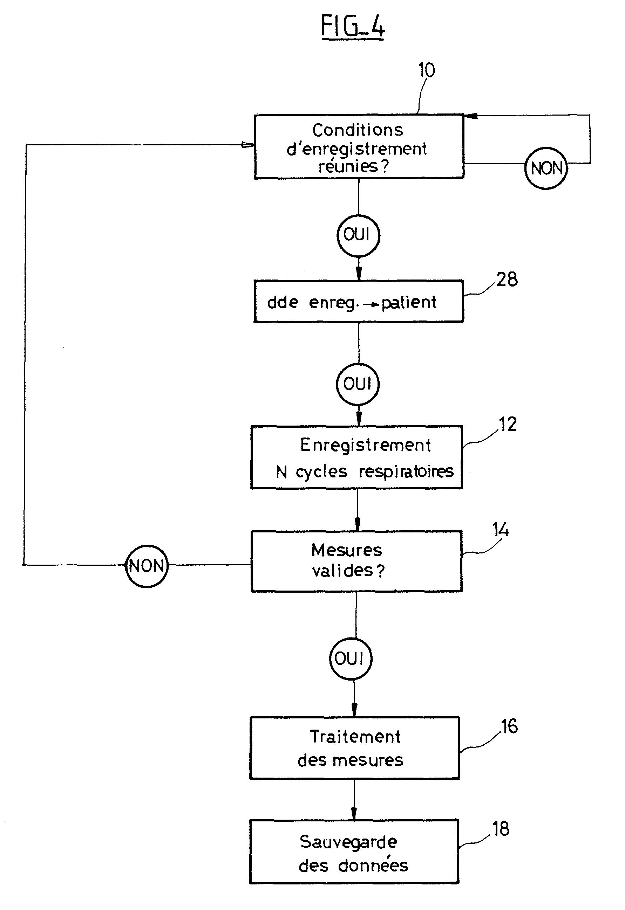

La Figure 4 illustre un autre mode de mise en oeuvre encore, dans lequel le dispositif enregistre à intervalles réguliers les cycles respiratoires, dans le cadre d'un suivi de l'état du patient. Mais à la différence de la figure 1, les cycles respiratoires sont ici des cycles forcés, et une participation active du patient est donc nécessaire.

Après que les conditions d'enregistrement aient été réunies (étape 10, comme dans le cas de la Figure 2) le patient déclenche l'enregistrement (étape 28).

Une première solution consiste à utiliser des moyens de télétransmission depuis l'implant vers un dispositif externe, par exemple un dispositif de *"home monitoring".* Une fois couplé (par télémétrie) à l'implant, le dispositif externe demande au patient d'effectuer dans un délai fixé un cycle d'inspiration/expiration forcées. Dès la confirmation de l'accord du patient, l'implant enregistre en continu des cycles respiratoires successifs (étape 12) et définit le cycle respiratoire forcé comme étant par exemple le cycle respiratoire présentant la plus grande amplitude crête-à-crête. Si ce cycle est conforme (étape 14, comme dans la Figure 2), alors il est analysé (étape 16) et mémorisé (étape 18).

Une autre solution, qui présente l'intérêt de ne nécessiter aucun dispositif externe, consiste à définir une séquence respiratoire spécifique dont le "profil" sera détecté par l'implant et interprété comme une demande d'enregistrement d'un cycle respiratoire forcé. La séquence peut être par exemple une séquence fixe "codée" du type trois cycles rapides à faible amplitude suivis du cycle d'inspiration/expiration forcée à enregistrer et analyser. Cette solution présente l'avantage de ne pas nécessiter de dialogue entre l'implant et un dispositif externe, mais ne permet pas de réitérer la mesure si l'enregistrement est jugé non utilisable, à moins d'envisager par exemple une confirmation de la validité de la mesure par un signal sonore émis par l'implant.

## Revendications

1. Un dispositif médical implantable actif, comprenant :
- des moyens de mesure de l'activité respiratoire du patient, aptes à recueillir un signal d'impédance transthoracique fonction des variations instantanées du volume pulmonaire,
**caractérisé en ce qu'**il comprend en outre :
- des moyens spirométriques, aptes à produire, à partir du signal d'impédance transthoracique recueilli sur au moins un cycle respiratoire, des couples de valeurs formés à partir :
• d'une part du volume pulmonaire instantané (V),
• d'autre part du débit pulmonaire instantané (dV/dt), représenté par la dérivée dudit volume pulmonaire,
pour produire à partir de ces couples de valeurs une caractéristique spirométrique (S) définie dans un espace débit vs. volume sans dimension temporelle.

2. Le dispositif de la revendication 1, comprenant en outre :
- des moyens (16) d'analyse de la caractéristique spirométrique produite, aptes à déduire de cette caractéristique au moins un paramètre d'état pulmonaire du patient.

3. Le dispositif de la revendication 2, comprenant en outre :
- des moyens de diagnostic (16) associés aux moyens d'analyse et aptes à opérer une comparaison entre, d'une part, une valeur de référence prédéterminée et, d'autre part, ledit paramètre d'état pulmonaire ou la variation dans le temps dudit paramètre d'état pulmonaire, et à produire un indicateur diagnostique en fonction du résultat de cette comparaison.

4. Le dispositif de la revendication 2, dans lequel les moyens d'analyse comprennent des moyens de recherche de points remarquables de la caractéristique spirométrique.

5. Le dispositif de la revendication 2, dans lequel les moyens d'analyse comprennent des moyens de modélisation linéaire ou curviligne de la caractéristique spirométrique.

6. Le dispositif de la revendication 1, comprenant en outre :
- des moyens (10) de validation *a priori,* aptes à déterminer l'existence d'au moins une condition d'état prédéterminée lors du recueil du signal d'impédance transthoracique, et à inhiber la mise en oeuvre des moyens spirométriques en cas d'absence de ladite condition d'état.

7. Le dispositif de la revendication 6, dans lequel ladite au moins une condition d'état prédéterminée est une condition du groupe comprenant : une plage de valeurs de fréquence cardiaque du patient ; un état de repos du patient ; et une plage horaire.

8. Le dispositif de la revendication 3, comprenant en outre :
- des moyens (14) de validation *a posteriori,* aptes à déterminer la conformité, ou non, de la caractéristique produite par les moyens spirométriques à au moins un critère prédéterminé, et à inhiber la mise en oeuvre des moyens de diagnostic en cas de non-conformité.

9. Le dispositif de la revendication 8, dans lequel ledit au moins un critère prédéterminé est un critère du groupe comprenant : le caractère fermé de la courbe décrivant la caractéristique spirométrique sur un cycle respiratoire ; et la stabilité de la caractéristique spirométrique sur une pluralité de cycles respiratoires successifs.

10. Le dispositif de la revendication 1, comprenant en outre :
- des moyens (20 ; 28) d'activation sélective des moyens spirométriques, opérant en réponse à un signal de commande externe.

11. Le dispositif de la revendication 10, dans lequel le signal de commande externe est un signal du groupe comprenant : un ordre externe transmis au dispositif par télémétrie ; et une séquence de cycles respiratoires spécifiques produits par le patient et détectés par les moyens spirométriques.
